# EUROPEAN PATENT SPECIFICATION

(11) **EP 2 434 271 B1**
(45) Date of publication and mention of the grant of the patent: **26.08.2015**
(21) Application number: 09784122.5
(22) Date of filing: 18.05.2009
(51) Int. Cl.: G01N 5/04, G01N 25/22, G01N 33/22

(54) **DEVICE FOR AUTOMATIC IN-LINE MEASUREMENT OF MASS LOSS BY CALCINATION AND THERMAL DECOMPOSITION OF SOLID PARTICLES**
VORRICHTUNG ZUR AUTOMATISCHEN IN-LINE-MESSUNG DES MASSEVERLUSTS DURCH KALZINIERUNG UND THERMOLYSE VON FESTSTOFFTEILCHEN
APPAREIL POUR LA MESURE AUTOMATIQUE EN LIGNE DE LA PERTE DE MASSE PAR CALCINATION ET DÉCOMPOSITION THERMIQUE DE PARTICULES SOLIDES

(43) Date of publication of application: 28.03.2012
(73) Proprietor: Inerco, Ingeniería, Tecnología y Consultoría, S.A., 41092 Sevilla (ES)
(72) Inventor: DELGADO LOZANO, Miguel A., E-41092 Sevilla (ES); TOVA HOLGADO, Enrique, E-41092 Sevilla (ES); REYES VALLE, Mariano, E-41092 Sevilla (ES); RODRIGUEZ BAREA, Francisco, E-41092 Sevilla (ES); CAÑADAS SERRANO, Luis, E-41092 Sevilla (ES); CORTES GALEANO, Vicente, E-41092 Sevilla (ES)
(74) Representative: Pons Ariño, Angel
(86) International application number: PCT/ES2009/070166
(87) International publication number: WO 2010/133715

(56) References cited:
- EP-A1- 1 413 873
- NL-A- 8 601 697
- DATABASE EPODOC EUROPEAN PATENT OFFICE, THE HAGUE, NL; 19 September 2007 (2007-09-19), XP002569201 & CN 200 950 111 Y (NANJING DALU ZHONGDIAN TECHNOL [CN]) 19 September 2007 (2007-09-19)
- DATABASE EPODOC EUROPEAN PATENT OFFICE, THE HAGUE, NL; 8 October 2008 (2008-10-08), XP002569202 & CN 201 130 130 Y (NANJING GUOSHENG TECHNOLOGY CO [CN]) 8 October 2008 (2008-10-08)

## Description

### OBJECT OF THE INVENTION

The invention refers, as expressed in the title of this report, to an apparatus for the online measurement of loss on ignition and thermal decomposition (pyrolysis) of solid particles automatically extracted from a duct through which they are transported by a gas current.

### FIELD OF APPLICATION

The present invention is of application in any industry in which the determination of loss on ignition and/or thermal decomposition (pyrolysis) is required in a sample of solid particles with a view to monitoring and optimising the process or even for quality control in a specific powder product.

Loss on ignition (LOI) is the reduction in mass expressed as a percentage in a sample of solid particles when heated at a controlled temperature in an oxidising environment. This parameter is usually related to the fixed carbon content of the sample of particles.

When the controlled rise in temperature takes place in the absence of oxygen, thermal decomposition or pyrolysis occurs, with the loss of mass under these conditions being related to the volatile material content in the solid particles sample.

The field of application of the invention is particularly applicable to boilers or ovens using solid fuels (coal, biomass), powdered or on fluidized beds, in which the unburned materials content of ashes is determined (solid residue of fuel combustion) by means of the determination of LOI.

Also, and within the same environment, the present invention enables the automated determination by means of the same loss on ignition parameter of the ash content of the fuel feeding the boiler, as well as the volatile material content in particular conditions of temperature increase and absence of oxygen.

These parameters are periodically measured in the laboratory for the quality control of the solid fuel.

### BACKGROUND OF THE INVENTION

One of the most important parameters for defining the performance of a solid fuel (coal, biomass) fired boiler or oven is the unburned materials content in the ash generated as a combustion residue. The control of this parameter is vital for establishing operational strategies of fuel consumption optimisation and, in consequence, CO₂ emissions.

The benefits of measuring this parameter additionally relate to the possibility of adequate monitoring of the characteristics of the ash with a view to its sale as an additive for the cement industry and also for defining limits for operational strategies for the control of NOₓ.

This important parameter has traditionally been determined by manual extraction of daily ash samples, normally retrieved from the pneumatic transport ducts leading from the electrofilter ash collection hoppers to the silo, and their subsequent analysis in the laboratory. This analysis is generally performed following procedures defined in various standards such as ISO 1171 "Solid mineral fuel-Determination of ash" or its equivalents in other countries such as the UNE 32-004-84 standard in Spain "Combustibles minerales sólidos-Determinación de cenizas" or ASTM C311 - 07 "Standard Test Methods for Sampling and Testing Fly Ash or Natural Pozzolans for Use in Portland-Cement Concrete". The procedure defined in these standards, very similar in all cases, consists of weighing the cold crucible in an analytical balance, weighing the crucible filled with the ash sample, heating to a specific temperature until constant weight (815 ºC in ISO and 750 ºC in ASTM or other temperature sufficient to cause thermal decomposition of the solid sample in oxidising conditions), cooling in a desiccator and weighing the crucible with the heated sample.

Additionally, these types of determination by controlled increase in temperature are applied to the determination of ash content in solid fuels, following methodologies and standards of thermal decomposition under oxidising conditions that are very similar to those referred to above for the determination of unburned material in ash, although recording, in this case of the measurement of ash content, the weight of residue produced instead of the loss in weight.

In the same context, determination of loss of weight after controlled temperature rise is of common application, although in non-oxidising conditions, for the determination of volatile material content in solid fuels. Examples of these determinations are those referred to in the ISO 562, UNE 32-019-84 or ASTM D 3175 standards. The procedure established in these standards is very similar to that defined for the determination of LOI, with the difference that a lid is placed over the crucible during the heating process and there is a specific heating programme (temperature and time) for each fuel.

Currently, there are automated laboratory equipment for simplifying the analysis process, minimising operator manipulation, fundamentally as regards the processes of weighing and heating. The high use of these equipment has implied the development of a specific standard regarding their design and use such as ASTM D5142 "Standard test method for proximate analysis of coal and coke by instrumental procedures" for performing the rapid analysis of coals by automated laboratory equipment. Examples of such equipment are the commercial systems Hot Foil LOI Instrument from FERCo or the MAC 400 from LECO.

Similarly, the patent US 4,846,292 describes an automated apparatus for the measurement of unburned materials in a number of samples loaded manually on a series of receptacles, which are analysed sequentially.

Also, there are laboratory equipment that determine the carbon content of ash samples by heating a known weight and measuring the CO₂ generated. This measurement is very similar quantitatively to the LOI value as the majority of unburned material in ash is carbon. These equipment often provide an element analysis of the sample, fundamentally carbon, hydrogen and nitrogen, as per the standard, ASTM D5373 "Standard Test Methods for Instrumental Determination of Carbon, Hydrogen, and Nitrogen in Laboratory Samples of Coal".

In all the cases described previously, the participation of an operative is necessary in the phase of extracting the sample and placing it into the automated laboratory analyser, as well as of its subsequent removal after the measurement.

This form of operation limits the monitoring of these important parameters to, in the majority of cases, a single daily value that is obtained on the day following the extraction of the sample. This makes it impossible to perform any effective adjustment on the combustion conditions (as a result of the change in unburned material in the ash or in the properties of the fuel).

As regards the particular case of the measurement of unburned material in ash, a variety of online equipment has been developed over the last few years for the measurement of this parameter with response times that are adequate for the implementation of effective control actions over the process. Generally these constitute a system for automatically taking samples, connected to the transport duct, and a system for the analysis of the extracted ash. In all these cases, the equipment is based on indirect measurement methods (capacitive infrared or microwave sensors) that are significantly susceptible to variations in the characteristics of the ash. This leads to significant uncertainly over the values provided in scenarios of changes in fuel, which are becoming increasingly frequent.

Finally it is also known document NL8601697 which discloses an apparatus for the automated online measurement of Loss On Ignition and thermal decomposition of solid particles drawn from a duct in which they are being transported in a gas current, comprising an oven provided with an upper lid and a lower lid and with orifices for the circulation of external air, a crucible for the collection and heating of the particles from the collection chamber, an analytical balance for weighing the crucible, a vertical discharge duct connecting the collection chamber to the crucible, a rigid rod linked to the crucible which penetrates the lower lid of the oven and is associated with the analytical balance, so that said analytical balance supports at least the crucible and the rod maintaining the vertical position of the crucible.

### DESCRIPTION OF THE INVENTION

The present invention refers to an apparatus for the online measurement of the Loss on Incineration of solid particles, of direct application to the determination of unburned material in ash and in the determination of volatile material and ash in fuel in solid fuel boilers and ovens such as those in coal-fired thermoelectric plants.

The basic principles of measurement by the equipment are those of the ISO 1171 standard for the determination of ash (or unburned material) and the UNE 32-019-84 standard for the determination of volatile material, both referring to a manual determination of the parameters, although the processes have been adapted to the conditions of automated equipment installed close to the duct. It therefore combines the exactness of equipment based on the reference standard (therefore not sensitive to changes in the nature and physico-chemical properties of the ash) with the speed of response of online systems connected to the process.

The equipment in question enables obtaining a totally automated measurement, without requiring the participation of operatives. It comprises the means necessary for the extraction of a sample of ash from a duct where they are being transported in a gas current; the weighing of an aliquot to be analysed; its heating at a controlled temperature in accordance with a specific programme (for example at 815 ºC as described in the ISO 1171 standard for the measurement of ash content or unburned material, or at any other temperature or thermal profiles at which it is desired to characterise the thermal decomposition or loss on ignition of the aliquot) in the presence or absence of oxygen depending on the parameter to be measured; its weighing after the heating process; the calculation of the loss in weight; the elimination of the analysed sample to the duct from which it came; and the cleaning a preparation of the system for a new cycle.

The fundamental characteristic of the equipment is its ability to place the particle sample in a recipient or cylindrical crucible, which is continuously inside an oven, preferably electric, with temperature control, as well as eliminating the heated sample to enable the start of a new measurement cycle; all these steps being performed totally automatically. To achieve this, the equipment has a duct that, penetrating the oven through the top, terminates, preferably in the form of a dome, at the height of the upper part of the crucible. The sample enters the oven under gravity through the previously mentioned duct and is deposited in the crucible where it is subjected to heating. The crucible is supported by a rod that, without contact with the walls of the oven, penetrates through the oven bottom, and rests on an analytical balance. In this way, the change in weight of the sample to be analysed is measured continuously from its entry in the oven to the end of the heating, detected by constant weight. After the analysis, the assembly comprising the balance, the supporting rod and the crucible is given a linear movement, in the direction of the axis of the oven, in such as way as to enable the circular base of the crucible to approach the discharge section of the dome of the duct, through which the sample entered. Simultaneously with approach of the crucible to the dome, the equipment provides suction through entry duct for the total elimination of the heated sample. The air necessary for heating in the presence of oxygen enters the oven through the free space between the lower lid of the oven and the rod supporting the crucible, circulates through the interior by natural draw and exits through an orifice made in the upper lid of the oven. For heating in the absence of oxygen, and therefore for the measurement of volatile material, the equipment has the possibility of placing a lid over the crucible to avoid the entry of air. This lid preferably has the form of a circular corona and is mounted in such a way that it is penetrated through its internal circumference by the duct through which the particles gain access to the interior of the oven. The lid can move freely in the vertical direction in such a way that, by its own weight, it rests on the dome of the entry duct through for the particles. The ability to move the crucible in the vertical direction enables its positioning in such a way as to bring its rim surface in contact with the lid, in this way preventing the entry of air. During the process of elimination of the sample after the analysis, the raising of the crucible displaces the lid upwards with respect to its position supported on the duct, enabling the entry of air for cleaning generated by the suction.

The configuration described above confers on the present invention a fundamental quality with respect to the accuracy of the measurement. The fact that the crucible is always inside the oven at a constant temperature and that a continuous measurement of sample weight is available from its entry into the crucible avoids the process of cooling, before weighing, after heating. This aspect is especially important as the variations of humidity experienced by the crucible during temperature changes can be of the same order or higher than the change in mass of the sample analysed. For this reason, in standard laboratory procedures, crucibles must be kept in desiccators for a time before and after heating, so that the weight measurements are performed under the same conditions of equilibrium with respect to the humidity of the crucible (the laboratory temperature is often kept constant). This aspect is more difficult to control in automated equipment, where the cycles of cooling can introduce uncertainty associated with the various ambient temperature and humidity conditions during the weight estimations.

Also, the continuous weighing of the sample during the heating process enables the identification of the moment of constant weight (end of the analysis), thereby preventing the performance of successive confirmatory weight measurements, and optimising in this way the response time of the equipment.

### DESCRIPTION OF THE FIGURES

In order to complement the description and with the aim of improving understanding of the characteristics of the invention, a series of illustrative and non-limiting figures is appended:
Figure 1 shows a schema of the apparatus assembly connected to the tubing through which the particles are transported in a gas current. In this figure, the crucible in the interior of the oven is shown in position during the phase of loading the sample to be analysed. This coincides with the position during the phase of heating in the absence of oxygen in the case of using the equipment for the measurement of volatile materials content.
Figure 2 shows the position of the crucible in the oven during the phases of weighing and heating in an oxidising atmosphere for the measurement of unburned materials content or ash content.
Figure 3 shows the position of the crucible in the oven during the phase of elimination of the analysed sample.

### PREFERRED EMBODIMENT OF THE INVENTION

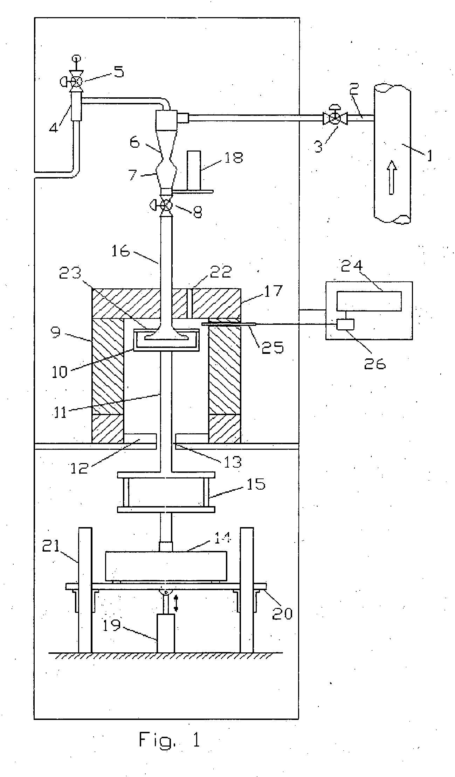

Figure 1 shows a schema of one possible embodiment of the invention that is the object of the patent. The configuration of the equipment enables the determination of loss of weight both in oxidising condition and in non-oxidising conditions.

In the example illustrated, the equipment for the online measurement of Loss On Ignition and thermal decomposition is connected to a pneumatic particle transport duct (1) through transport tubing (2) where the connection has an automated shutoff valve (3). The equipment uses a jet pump (4), supplied with compressed air through an automated shutoff value (5), to suck a biphasic current composed of a mixture of particles to be analysed and the transporting air. The particles extracted are separated by means of a cyclone separator (6) and collected in a chamber (7), which is isolated at the lower end by an automated shutoff value (8).

The cyclone separator (6), the chamber (7) and the valve (8) are all arranged linearly over the electric oven (9), the temperature of which is controlled depending on the analysis to be performed. The oven (9) has a cylindrical crucible (10) inside, of a sufficient volume to collect the particles to be analysed. This crucible (10) is supported on a rod (11) that penetrates the oven through the lower lid (12) through an orifice (13) of a diameter that is greater than that of the rod (11) itself. In addition to providing support, the main function of the rod (11) is to transmit the weight of the sample to be analysed to a high precision balance (14) placed below the oven (9). To prevent heat transmission to the balance (14), the rod (11) is connected to a refrigerator (15) composed of two parallel flat plates connected by two separators. The refrigerator (15) is firmly fixed to the balance (14) in such a way that the verticality of the assembly formed by the crucible (10), the rod (11) and the refrigerator (15) itself is guaranteed and no contact exists between the balance pan (14) and the components of the oven (9).

The discharge of the particle sample from the chamber (7) to the crucible (10) takes place under gravity through a vertical duct (16), connected at one of its ends to the isolation value (8) and provided with a dome at the other end, which penetrates the oven through the upper lid (17) made of insulating material. The fall of the particles is encouraged by a pneumatic vibrator (18) acting on the chamber (7) and which is activated during the opening of the isolation valve (8). The vertical duct (16) is also arranged vertically aligned with the axis of the rod (11) and the maximum diameter of the dome is between 2 and 4 millimetres less than the internal diameter of the crucible (10).

The distance of the crucible (10) from the point of discharge from the vertical duct (16) is variable depending on the phase of the measurement process. To achieve this, the balance (14) and all its load, including the refrigerator (15), the rod (11) and the crucible (10) can be moved vertically by means of a vertical displacement mechanism (19, 20, 21) comprising at least a linear actuator (19) linked to a platform (20) that serves to support the whole of the assembly. The verticality of movement, for the purpose of avoiding lateral contact of the balance pan (14) with the oven (9) walls, is ensured by the guides (21) of this mechanism (19, 20, 21), which penetrate the platform (20).

During the phase of loading the sample into the crucible (10), this is elevated up to half its height so that the discharge point of the vertical duct (16) is introduced into the interior, as shown in Figure 1.

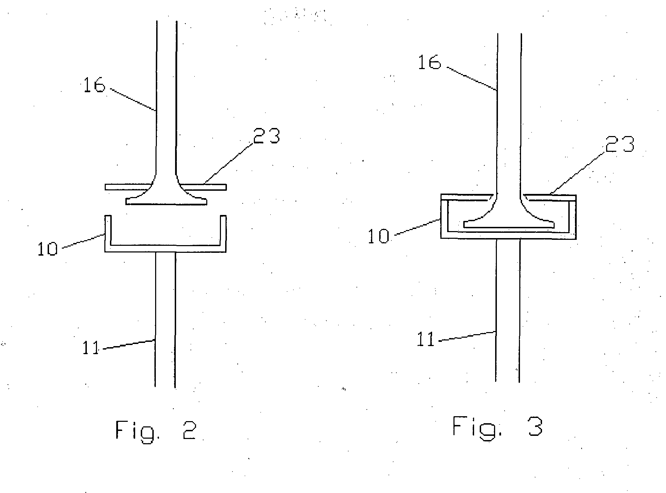

During the phases of reading the weight and heating in oxidising atmosphere, the crucible (10) is positioned so that its rim section is a few millimetres away from the discharge point of the vertical duct (16), as shown in Figure 2.

More specifically, the vertical displacement mechanism (19, 20, 21) is arranged to vary the vertical distance between the discharge opening of the vertical duct (16) and the internal base of the crucible (10) in at least three different vertical positions: a first position in which the distance is less than 2 millimetres, a second position in which the distance is equal to or greater than 2 millimetres and less than the height of the crucible (10) and a third position in which the distance is greater than the height of the crucible (10).

The circulation of air in the oven (9), necessary to ensure the presence of oxygen for the ignition reaction, is caused by natural drawing through an orifice (22) made in the upper lid (17) of the oven. Renewal of air occurs through an annular space between the rod (11) and the orifice (13) of larger diameter through which the rod penetrates the lower lid (12) of the oven.

In order to perform heating in non-oxidising conditions, the equipment is provided with a lid (23) in the form or a circular corona with its external diameter being equal to or greater than the exterior diameter of the crucible (10) and its internal diameter a few millimetres greater than the exterior diameter of the vertical discharge duct (16) for the particles, in the not-domed zone. This lid (23) is penetrated by the vertical duct (16) and rests on the domed surface of the duct by its own weight. During the heating phase in an analysis of volatile materials content, the crucible (10) is raised until its rim surface coincides with the lower surface of the lid (23), as shown in Figure 1. This moment is detected by the increase in weight recorded on the balance pan (14) as it is lightly loaded with the lid (23). The absence of air requires the closure of the isolating valve (8) during the heating.

In this case, the lid (23), the crucible (10) and the discharge duct (16) are sized in such a way that in the referred first position, the lid (23) rests supported on the crucible (10) without making contact with the vertical duct (16), in the referred second position, the lid (23) makes contact with the upper section of the crucible (10) and in the referred third position, the lid (23) is supported on the domed surface of the vertical duct (16).

For the determination of Loss On Ignition in an oxidising atmosphere, the equipment does not require the use of the lid (23) of the crucible (10) as the presence of oxygen does not affect the functioning of the equipment for this purpose.

To eliminate the sample after analysis, suction is provided through the vertical duct (16) by supplying compressed air to the jet pump (4), keeping valve (3) closed. Simultaneously to this suction, the crucible (10) is elevated until its internal base reaches the discharge point of the vertical duct (16), as shown in Figure 3. Once the sample has been eliminated, the crucible (10) is taken to the position of Figure 1 for loading a new sample and the start of a new cycle.

Automated control of the equipment is achieved through a control system (24), usually a PLC, that governs the opening and closure of the valves (3), (5) and (8), the activation of the linear actuator (19) and the functioning of the balance (14). It also manages the temperature control (25, 26) of the oven (9) comprising a temperature probe (25) and a control module (26) to which it is connected. It additionally performs the calculations of loss of weight on heating and has the means to send the value by remote control or to show it on a data visualisation display.

## Claims

1. Apparatus for the automated online measurement of Loss On Ignition and thermal decomposition of solid particles drawn from a duct (1) in which they are being transported in a gas current, comprising:
an oven (9) provided with an upper lid (17) and a lower lid (12) and with orifices (13, 22) for the circulation of external air,
a crucible (10) for the collection and heating of the particles from a collection chamber (7),
an analytical balance (14) for weighing the crucible (10),
a vertical discharge duct (16), connecting the collection chamber (7) to the crucible (10) constantly held inside the oven (9), said discharge duct penetrates the upper lid (17) of the oven (9) and is furnished with a lower discharge opening situated close to the crucible (10),
a rigid rod (11) linked to the crucible (10), the axis of which is aligned with the axis of the duct (16), which penetrates the lower lid (12) of the oven and is associated with the analytical balance (14), so that said analytical balance (14) supports at least the crucible (10) and the rod (11) maintaining the vertical position of the crucible (10),
**characterised in that** it additionally comprises:
a cyclone separator (6) into which the solid particles are introduced supplied with compressed air through an automated shutoff valve (5) and with a tangential input connected to the duct (1) the cyclone separator (6) and the duct (1) being connected through a transport tubing (2) where the connection has an automated shutoff valve (3), the cyclone separator (6) being also linked to the collection chamber (7), the collection chamber being located below the cyclone separator and isolated at its lower end by an automated shutoff valve (8),
a jet pump (4) connected to the cyclone separator (6) the jet pump being arranged to cause suction and facilitate the extraction of the sample from the duct (1) towards the cyclone separator (6) or the aspiration of the residue from the crucible (10) after the heating,
a vertical displacement mechanism (19, 20, 21) of the balance (14) and its pan, arranged to control the vertical positioning of the internal base of the crucible (10) with respect to the discharge opening of the duct (16), and
a control system (24) adapted to automatically manage the discharge of particles to the crucible (10) and the extraction of the heated particles from the crucible (10) said control system further being adapted to govern:
the opening and closure of the shutoff valves (3, 5, 8),
the positioning of the vertical displacement mechanism (19, 20, 21) of the balance (14),
the functioning of the balance (14),
the temperature control (25, 26) of the oven (9),
and to perform the calculations of loss of weight on heating.

2. Apparatus for the automated online measurement of Loss On Ignition and thermal decomposition of solid particles of claim 1, **characterised in that** the lower opening of the discharge duct (16) is domed.

3. Apparatus for the automated online measurement of Loss On Ignition and thermal decomposition of solid particles of claim 2, **characterised in that** in additionally comprises a lid (23) in the form of a corona adapted to close the upper opening of the crucible (10), and its internal diameter is slightly greater than the exterior diameter of the vertical duct (16) in its cylindrical not-domed section, with this lid (23) being penetrated by the vertical duct (16) without any type of binding and adapted to rest by its own weight on the domed section of the vertical duct (16).

4. Apparatus for the automatic online measurement of Loss On Ignition and thermal decomposition of solid particles of claim 1, **characterised in that** the crucible (10) is cylindrical.

5. Apparatus for the automatic online measurement of Loss On Ignition and thermal decomposition of solid particles of claims 3 and 4, **characterised in that** the coronal shape of the lid (23) is circular and its external diameter is equal to or greater than the external diameter of the crucible (10).

6. Apparatus for the automatic online measurement of Loss On Ignition and thermal decomposition of solid particles of claim 1, **characterised in that** the collection chamber (7) is provided with a lower route connected to an automated shutoff valve (8) and a vibration mechanism (18).

7. Apparatus for the automatic online measurement of Loss On Ignition and thermal decomposition of solid particles of claim 1, **characterised in that** one of the orifices (22) of the oven (9) is on the upper lid (17).

8. Apparatus for the automatic online measurement of Loss On Ignition and thermal decomposition of solid particles of claim 1, **characterised in that** one of the orifices (13) of the oven (9) is on the lower lid (12).

9. Apparatus for the automatic online measurement of Loss On Ignition and thermal decomposition of solid particles of claim 8, **characterised in that** the orifice (13) on the lower lid (12) of the oven (9) is penetrated by the rod (11) in such a way that it leaves an annular space free for the entry of air.

10. Apparatus for the automatic online measurement of Loss On Ignition and thermal decomposition of solid particles of claim 1, **characterised in that** the jet pump (4) is fuelled by compressed air.

11. Apparatus for the automatic online measurement of Loss On Ignition and thermal decomposition of solid particles of claim 1, **characterised in that** the vertical displacement mechanism (19, 20, 21) comprises a linear actuator (19) linked by its piston rod to a platform (20), on which the balance (14) rests, adapted to change the vertical distance between the opening of the vertical duct (16) and the internal base of the crucible (10) between at least three different positions: a first position in which the distance is less than 2 millimetres, a second position in which the distance is equal to or greater than 2 millimetres and less than the height of the crucible (10) and a third position in which the distance is greater than the height of the crucible (10).

12. Apparatus for the automatic online measurement of Loss On Ignition and thermal decomposition of solid particles of claim 11, **characterised in that** the vertical displacement mechanism (19, 20, 21) additionally comprises a least one guide (21) on which the platform (20) slides.

13. Apparatus for the automatic online measurement of Loss On Ignition and thermal decomposition of solid particles of claim 3 and 11, **characterised in that** the lid (23), the crucible (10) and the discharge duct (16) are sized in such a way that in the first position the lid (23) rests supported on the crucible (10) without making contact with the vertical duct (16), in the second position the lid (23) makes contact with the upper section of the crucible (10) and in the third position the lid (23) is supported on the domed surface of the vertical duct (16).

14. Apparatus for the automatic online measurement of Loss On Ignition and thermal decomposition of solid particles of claim 1, **characterised in that** it additionally comprises a refrigerator (15) situated between the balance (14) and the rod (11).

15. Apparatus for the automatic online measurement of Loss On Ignition and thermal decomposition of solid particles of claim 1, **characterised in that** the means of temperature control (25, 26) of the oven (9) comprise a temperature probe (25) and a temperature controller module (26).

16. Apparatus for the automatic online measurement of Loss On Ignition and thermal decomposition of solid particles of claim 1, **characterised in that** the control system (24) has a data visualisation display.

17. Apparatus for the automatic online measurement of Loss On Ignition and thermal decomposition of solid particles of claim 1, **characterised in that** the control system (24) has means to transmit data by remote control.

## Patentansprüche

1. Vorrichtung zur automatischen In-line-Messung des Masseverlusts durch Kalzinierung und Thermolyse von Feststoffteilchen, die aus einem Kanal (1) angesaugt werden, in dem sie in einem Gasstrom transportiert werden, umfassend:
einen Ofen (9), der mit einem oberen Deckel (17) und einem unteren Deckel (12) sowie mit Öffnungen (13, 22) für die Zirkulation der Außenluft ausgestattet ist,
einen Schmelztiegel (10) für die Sammlung und Erhitzung der Teilchen aus einer Sammelkammer (7),
eine Analysewaage (14) zur Verwiegung des Schmelztiegels (10),
ein vertikaler Austragkanal (16), der die Sammelkammer (7) mit dem ständig im Ofen (9) verbleibenden Schmelztiegel (10) verbindet, wobei dieser Austragkanal den oberen Deckel (17) des Ofens (9) durchdringt und mit einer unteren, in der Nähe des Schmelztiegels (10) angeordneten Austragöffnung ausgestattet ist,
eine steife Stange (11), die mit dem Schmelztiegel (10) verbunden ist, wobei deren Achse mit der Achse des Kanals (16) ausgerichtet ist und die den unteren Deckel (12) des Ofens durchdringt und mit der Analysewaage (14) verknüpft ist, sodass diese Analysewaage (14) mindestens den Schmelztiegel (19) und die Stange (11) stützt, wobei die vertikale Position des Schmelztiegels (10) aufrechterhalten wird,
**dadurch gekennzeichnet, dass** diese zusätzlich Folgendes umfasst:
einen Zyklonabscheider (6), in den die Feststoffteilchen eingeführt werden, wobei dieser über ein automatisches Absperrventil (5) mit Druckluft versorgt wird und mit einem tangentialen, mit dem Kanal (1) verbundenen Eingang ausgestattet ist, wobei der Zyklonabscheider (6) und der Kanal (1) über eine Transportrohrleitung (2) verbunden sind, wobei die Verbindung über ein automatisches Absperrventil (3) verfügt, wobei der Zyklonabscheider (6) ebenfalls mit der Sammelkammer (7) verbunden ist, wobei sich die Sammelkammer unter dem Zyklonabscheider (6) befindet und an ihrem unteren Ende durch ein automatisches Absperrventil (8) isoliert ist,
eine Strahlpumpe (4), die mit dem Zyklonabscheider (6) verbunden ist, wobei die Strahlpumpe dazu angeordnet ist, einen Sog zu erzeugen und die Absaugung der Probe aus dem Kanal (1) in Richtung des Zyklonabscheiders (6) oder die Ansaugung der Rückstände aus dem Schmelztiegel (10) nach der Erhitzung zu erleichtern,
ein vertikaler Verdrängungsmechanismus (19, 20, 21) der Waage (14) und deren Schale, der dazu angeordnet ist, die vertikale Positionierung der internen Basis des Schmelztiegels (10) gegenüber der Austragöffnung des Kanals (16) zu steuern, und
ein Steuersystem (24), das dazu ausgelegt ist, automatisch den Austrag der Teilchen an den Schmelztiegel (10) und die Absaugung der erhitzten Teilchen aus dem Schmelztiegel (10) zu handhaben, wobei dieses Steuersystem weiterhin dazu ausgelegt ist, Folgendes zu steuern:
die Öffnung und Schließung der Absperrventile (3, 5, 8)
die Positionierung des vertikalen Verdrängungsmechanismus (19, 20, 21) der Waage (14),
die Arbeitsweise der Waage (14),
die Temperaturregelung (25, 26) des Ofens (9),
sowie die Berechnungen des Masseverlusts bei der Erhitzung durchzuführen.

2. Vorrichtung zur automatischen In-line-Messung des Masseverlusts durch Kalzinierung und Thermolyse von Feststoffteilchen nach Anspruch 1, **dadurch gekennzeichnet, dass** die untere Öffnung des Austragkanals (16) gewölbt ist.

3. Vorrichtung zur automatischen In-line-Messung des Masseverlusts durch Kalzinierung und Thermolyse von Feststoffteilchen nach Anspruch 2, **dadurch gekennzeichnet, dass** diese zusätzlich einen Deckel (23) in Form einer Korona umfasst, der dazu ausgelegt ist, die obere Öffnung des Schmelztiegels (10) zu schließen, wobei dessen Innendurchmesser etwas größer als der Außendurchmesser des vertikalen Kanals (16) an dessen zylinderförmigen, nicht gewölbten Abschnitt ist, wobei dieser Deckel (23) von dem vertikalen Kanal (16) ohne irgendeine Art von Bindung durchdrungen wird und dazu ausgelegt ist, aufgrund seines Eigengewichts auf dem gewölbten Abschnitt des vertikalen Kanals (16) zu ruhen.

4. Vorrichtung zur automatischen In-line-Messung des Masseverlusts durch Kalzinierung und Thermolyse von Feststoffteilchen nach Anspruch 1, **dadurch gekennzeichnet, dass** der Schmelztiegel (10) zylinderförmig ist.

5. Vorrichtung zur automatischen In-line-Messung des Masseverlusts durch Kalzinierung und Thermolyse von Feststoffteilchen nach Anspruch 3 und 4, **dadurch gekennzeichnet, dass** die Koronaform des Deckels (23) kreisförmig ist und dessen Außendurchmesser gleich oder größer als der Außendurchmesser des Schmelztiegels (10) ist.

6. Vorrichtung zur automatischen In-line-Messung des Masseverlusts durch Kalzinierung und Thermolyse von Feststoffteilchen nach Anspruch 1, **dadurch gekennzeichnet, dass** die Sammelkammer (7) mit einer unteren Route ausgestattet ist, die mit einem automatischen Absperrventil (8) und einem Vibrationsmechanismus (18) verbunden ist.

7. Vorrichtung zur automatischen In-line-Messung des Masseverlusts durch Kalzinierung und Thermolyse von Feststoffteilchen nach Anspruch 1, **dadurch gekennzeichnet, dass** eine der Öffnungen (22) des Ofens (9) sich in dem oberen Deckel (17) befindet.

8. Vorrichtung zur automatischen In-line-Messung des Masseverlusts durch Kalzinierung und Thermolyse von Feststoffteilchen nach Anspruch 1, **dadurch gekennzeichnet, dass** eine der Öffnungen (13) des Ofens (9) sich in dem unteren Deckel (12) befindet.

9. Vorrichtung zur automatischen In-line-Messung des Masseverlusts durch Kalzinierung und Thermolyse von Feststoffteilchen nach Anspruch 8, **dadurch gekennzeichnet, dass** die Öffnung (13) im unteren Deckel (12) des Ofens (9) von der Stange (11) auf eine solche Weise durchdrungen wird, dass diese einen ringförmigen Raum für den Einlass von Luft freilässt.

10. Vorrichtung zur automatischen In-line-Messung des Masseverlusts durch Kalzinierung und Thermolyse von Feststoffteilchen nach Anspruch 1, **dadurch gekennzeichnet, dass** die Strahlpumpe (4) mit von Druckluft betrieben wird.

11. Vorrichtung zur automatischen In-line-Messung des Masseverlusts durch Kalzinierung und Thermolyse von Feststoffteilchen nach Anspruch 1, **dadurch gekennzeichnet, dass** der vertikale Verdrängungsmechanismus (19, 20, 21) ein lineares Stellglied (19) umfasst, das über seine Kolbenstange mit einer Plattform (20) verbunden ist, auf der die Waage (14) ruht, dazu ausgelegt, den vertikalen Abstand zwischen der Öffnung des vertikalen Kanals (16) und der internen Basis des Schmelztiegels (10) auf mindestens drei unterschiedlichen Positionen zu ändern: eine erste Position, bei welcher der Abstand weniger als 2 Millimeter beträgt, eine zweite Position, bei welcher der Abstand gleich oder größer als 2 Millimeter und geringer als die Höhe des Schmelztiegels (10) ist, und eine dritte Position, bei welcher der Abstand größer als die Höhe des Schmelztiegels (10) ist.

12. Vorrichtung zur automatischen In-line-Messung des Masseverlusts durch Kalzinierung und Thermolyse von Feststoffteilchen nach Anspruch 11, **dadurch gekennzeichnet, dass** der vertikale Verdrängungsmechanismus (19, 20, 21) zusätzlich mindestens eine Führung (21) umfasst, auf der die Plattform (20) gleitet.

13. Vorrichtung zur automatischen In-line-Messung des Masseverlusts durch Kalzinierung und Thermolyse von Feststoffteilchen nach Anspruch 3 und 11, **dadurch gekennzeichnet, dass** der Deckel (23), der Schmelztiegel (10) und der Austragkanal (16) auf eine solche Weise bemessen sind, dass der Deckel (23) in der ersten Position auf dem Schmelztiegel (10) ruht, ohne den vertikalen Kanal (16) zu berühren, dass der Deckel (23) in der zweiten Position den oberen Abschnitt des Schmelztiegels (10) berührt und dass der Deckel (23) in der dritten Position von der gewölbten Oberfläche des vertikalen Kanals (16) gestützt wird.

14. Vorrichtung zur automatischen In-line-Messung des Masseverlusts durch Kalzinierung und Thermolyse von Feststoffteilchen nach Anspruch 1, **dadurch gekennzeichnet, dass** diese zusätzlich eine Kühlanlage (15) umfasst, die zwischen der Waage (14) und der Stange (11) angeordnet ist.

15. Vorrichtung zur automatischen In-line-Messung des Masseverlusts durch Kalzinierung und Thermolyse von Feststoffteilchen nach Anspruch 1, **dadurch gekennzeichnet, dass** die Mittel zur Temperaturregelung (25, 26) des Ofens (9) einen Temperaturfühler (25) und ein Temperaturregelmodul (26) umfassen.

16. Vorrichtung zur automatischen In-line-Messung des Masseverlusts durch Kalzinierung und Thermolyse von Feststoffteilchen nach Anspruch 1, **dadurch gekennzeichnet, dass** das Steuersystem (24) ein Datenvisualisierungsdisplay besitzt.

17. Vorrichtung zur automatischen In-line-Messung des Masseverlusts durch Kalzinierung und Thermolyse von Feststoffteilchen nach Anspruch 1, **dadurch gekennzeichnet, dass** das Steuersystem (24) Mittel zur ferngesteuerten Datenübertragung besitzt.

## Revendications

1. Appareil pour la mesure automatique en ligne de la perte de masse par calcination et décomposition thermique de particules solides tirées d'un conduit (1) dans lequel elles sont transportées dans un courant de gaz, comprenant :
un four (9) muni d'un couvercle supérieur (17) et d'un couvercle inférieur (12) et d'orifices (13, 22) pour la circulation de l'air extérieur,
un creuset (10) pour la collecte et le chauffage des particules d'un collecteur (7),
une balance analytique (14) pour peser le creuset (10),
un conduit de déchargement vertical (16), reliant le collecteur (7) au creuset (10) constamment maintenu à l'intérieur du four (9), ledit conduit de déchargement pénètre dans le couvercle supérieur (17) du four (9) et il est muni d'une ouverture de déchargement inférieure située près du creuset (10),
une tige rigide (11) reliée au creuset (10), dont l'axe est aligné avec l'axe du conduit (16), qui pénètre dans le couvercle inférieur (12) du four et qui est associé à la balance analytique (14), pour que ladite balance analytique (14) supporte au moins le creuset (10) et la tige (11) en maintenant la position verticale du creuset (10),
**caractérisé en ce qu'**il comprend en outre :
un séparateur à cyclone (6), dans lequel les particules solides sont introduites, alimenté en air comprimé via une soupape d'arrêt automatique (5) et avec une entrée tangentielle reliée au conduit (1), le séparateur à cyclone (6) et le conduit (1) étant connectés par un tube d'acheminement (2) où la connexion possède une soupape d'arrêt automatique (3), le séparateur à cyclone (6) étant également relié au collecteur (7), le collecteur étant situé en dessous du séparateur à cyclone et isolé à son extrémité inférieure par une soupape d'arrêt automatique (8),
une pompe à jet (4) connectée au séparateur à cyclone (6), la pompe à jet étant disposée pour provoquer une aspiration et faciliter l'extraction de l'échantillon depuis le conduit (1) en direction du séparateur à cyclone (6) ou l'aspiration des résidus depuis le creuset (10) après le chauffage,
un mécanisme de déplacement vertical (19, 20, 21) de la balance (14) et son plateau, disposé pour contrôler le positionnement vertical de la base interne du creuset (10) par rapport à l'ouverture de déchargement du conduit (16), et
un système de contrôle (24) adapté pour gérer automatiquement le déchargement des particules dans le creuset (10) et l'extraction des particules chauffées du le creuset (10), ledit système de contrôle étant en outre adapté pour gouverner :
l'ouverture et la fermeture des soupapes d'arrêt (3, 5, 8),
le positionnement du mécanisme de déplacement vertical (19, 20, 21) de la balance (14),
le fonctionnement de la balance (14)
le contrôle de la température (25, 26) du four (9),
et pour réaliser les calculs de perte de masse lors du chauffage.

2. Appareil pour la mesure automatique en ligne de la perte de masse par calcination et décomposition thermique de particules solides de la revendication 1, **caractérisé en ce que** l'ouverture inférieure du conduit de déchargement (16) est bombée.

3. Appareil pour la mesure automatique en ligne de la perte de masse par calcination et décomposition thermique de particules solides de la revendication 2, **caractérisé en ce qu'**il comprend en outre un couvercle (23) en forme de couronne adaptée pour fermer l'ouverture supérieure du creuset (10), et son diamètre intérieur est légèrement plus grand que le diamètre extérieur du conduit vertical (16) dans sa section cylindrique non bombée, avec ce couvercle (23) qui est pénétré par le conduit vertical (16) sans aucun type de liaison et adapté pour reposer sous l'effet de son propre poids sur la section bombée du conduit vertical (16).

4. Appareil pour la mesure automatique en ligne de la perte de masse par calcination et décomposition thermique de particules solides de la revendication 1, **caractérisé en ce que** le creuset (10) est cylindrique.

5. Appareil pour la mesure automatique en ligne de la perte de masse par calcination et décomposition thermique de particules solides des revendications 3 et 4, **caractérisé en ce que** la forme en couronne du couvercle (23) est circulaire et son diamètre extérieur est égal à ou plus grand que le diamètre extérieur du creuset (10).

6. Appareil pour la mesure automatique en ligne de la perte de masse par calcination et décomposition thermique de particules solides de la revendication 1, **caractérisé en ce que** le collecteur (7) est pourvu d'une voie inférieure reliée à une soupape d'arrêt automatique (8) et à un mécanisme de vibration (18).

7. Appareil pour la mesure automatique en ligne de la perte de masse par calcination et décomposition thermique de particules solides de la revendication 1, **caractérisé en ce que** l'un des orifices (22) du four (9) est sur le couvercle supérieur (17).

8. Appareil pour la mesure automatique en ligne de la perte de masse par calcination et décomposition thermique de particules solides de la revendication 1, **caractérisé en ce que** l'un des orifices (13) du four (9) est sur le couvercle inférieur (12).

9. Appareil pour la mesure automatique en ligne de la perte de masse par calcination et décomposition thermique de particules solides de la revendication 8, **caractérisé en ce que** l'orifice (13) sur le couvercle inférieur (12) du four (9) est pénétré par la tige (11) de telle façon qu'elle laisse un espace annulaire libre pour l'entrée d'air.

10. Appareil pour la mesure automatique en ligne de la perte de masse par calcination et décomposition thermique de particules solides de la revendication 1, **caractérisé en ce que** la pompe à jet (4) est chargée en air comprimé.

11. Appareil pour la mesure automatique en ligne de la perte de masse par calcination et décomposition thermique de particules solides de la revendication 1, **caractérisé en ce que** le mécanisme de déplacement vertical (19, 20, 21) comprend un actionneur linéaire (19) relié par sa tige de piston à une plateforme (20), sur laquelle la balance (14) repose, apte à changer la distance verticale entre l'ouverture du conduit vertical (16) et la base interne du creuset (10) entre au moins trois positions différentes : une première position dans laquelle la distance est inférieure 2 millimètres, une deuxième position dans laquelle la distance est égale ou supérieure à 2 millimètres et inférieure à la hauteur du creuset (10) et une troisième position dans laquelle la distance est supérieure à la hauteur du creuset (10).

12. Appareil pour la mesure automatique en ligne de la perte de masse par calcination et décomposition thermique de particules solides de la revendication 11, **caractérisé en ce que** le mécanisme de déplacement vertical (19, 20, 21) comprend en outre au moins un guide (21) sur lequel glisse la plateforme (20).

13. Appareil pour la mesure automatique en ligne de la perte de masse par calcination et décomposition thermique de particules solides des revendications 3 et 11, **caractérisé en ce que** le couvercle (23), le creuset (10) et le conduit de déchargement (16) sont dimensionnés de telle sorte que dans la première position le couvercle (23) repose soutenu par le creuset (10) sans être en contact avec le conduit vertical (16), dans la deuxième position le couvercle (23) est en contact avec la section supérieure du creuset (10) et dans la troisième position le couvercle (23) repose sur la surface bombée du conduit vertical (16).

14. Appareil pour la mesure automatique en ligne de la perte de masse par calcination et décomposition thermique de particules solides de la revendication 1, **caractérisé en ce qu'**il comprend en outre un réfrigérateur (15) situé entre la balance (14) et la tige (11).

15. Appareil pour la mesure automatique en ligne de la perte de masse par calcination et décomposition thermique de particules solides de la revendication 1, **caractérisé en ce que** les moyens de contrôle de la température (25, 26) du four (9) comprennent une sonde de température (25) et un module de contrôle de la température (26).

16. Appareil pour la mesure automatique en ligne de la perte de masse par calcination et décomposition thermique de particules solides de la revendication 1, **caractérisé en ce que** le système de contrôle (24) possède un dispositif d'affichage pour la visualisation des données.

17. Appareil pour la mesure automatique en ligne de la perte de masse par calcination et décomposition thermique de particules solides de la revendication 1, **caractérisé en ce que** le système de contrôle (24) possède des moyens pour transmettre des données par commande à distance.
